# EUROPEAN PATENT APPLICATION

(11) **EP 4 312 151 A1**
(43) Date of publication of application: **31.01.2024**
(21) Application number: 22187109.8
(22) Date of filing: 27.07.2022
(51) Int. Cl.: G06K 9/62, G06V 10/774, G06V 10/778, G06V 10/82, G06V 20/69

(54) **ARTIFICIAL INTELLIGENCE TRAINING SYSTEM FOR MEDICAL APPLICATIONS AND METHOD**

(71) Applicant: Siemens Healthcare GmbH, 91052 Erlangen (DE)
(72) Inventor: Marquardt, Gaby, 91353 Hausen (DE); Tietz, Christoph, 85521 Ottobrunn (DE); von Beuningen, Anja, 99085 Erfurt (DE)
(74) Representative: Siemens Healthineers Patent Attorneys

(57) **Abstract**

The invention relates to a training system and a training method for training an artificial neural network, in particular for medical applications. The training system comprises an image-processing device, which is configured to receive a set of acquired images, an artificial intelligence module, configured to implement an artificial neural network, and a controller device, configured to train the artificial neural network implemented by the artificial intelligence module, wherein the image-processing device is further configured to generate modified images based on a modification of the acquired images, wherein each of the modified images generated by the image-processing device contains less texture information than the respective acquired image from which they stem, and wherein the controller device is configured to receive a set of training images comprising at least part of the modified images and at least part of the acquired images and train an artificial neural network which, based on a classification scheme, is configured to output a probability distribution for each training image according to the classification scheme.

## Description

The present invention relates to a training system and method to train an artificial neural network, preferably convolutional neural networks, for medical applications. In particular, it relates to the augmentation of training data in order to improve the performance of artificial neural networks as classifiers.

The invention will, by way of example, be mostly applied to the fields of hematology and immunology, and in particular to the identification of white blood cells, but the principles of the invention have a broader scope.

In the medical diagnosis of diseases from blood analysis it is of the outmost importance to have a reliable counting of red blood cells, white blood cells and platelets. This necessarily entails a reliable identification of the blood constituents.

In blood diagnosis one routinely deals with stained blood samples, which in the form of blood smears can be examined with microscopes and eventually achieve a separation of the blood constituents and a counting thereof.

This procedure is time consuming and prone to errors. In the particular case of leucocytes, one of the main difficulties is that the canonical morphological features of each cell type are not unambiguous. In particular, the different maturation stages of the cells have an impact on their morphology and can be easily confused with other cell types. In the event of pathological cells the morphological differences can be even bigger. While this can be a smoking gun for disease diagnosis, at the same time it makes the classification of lymphocytes more difficult. In manual counting one should also take into account the human error as a source of uncertainty.

Cell counting is done in the recent years by using the ever evolving techniques of artificial intelligence, and in particular with the use of convolutional neural networks. One of the problems of artificial neural networks is that it is hard to control which features are underrepresented while training and which ones are overrepresented. This does not appear in particular applications but it is a generic characteristic of artificial neural networks.

This potential bias in the extraction of features by the artificial neural network during training is a source of potential misidentifications and affects the performance of the artificial neural networks. In the literature there have been mostly two directions to correct this bias. One is the addition of specially selected features to facilitate the identification and classification. The selection of these features for each application is, however, not easy, and neither it is its effective implementation in artificial neural networks. Another line of investigation has dealt with the addition of metadata to the medical image data. Information about the patient age or gender, or previous medical conditions, is added. Additionally, information extracted from the images can also be provided. For instance, in US 2019 0347467 A1, information about the cell size, excentricity of the cell nucleus, or presence of vacuoles can be added to each image. However, this requires an effort to generate this metadata, find storage space to save it and then manage it to improve the artificial neural network performance.

It is an object of the present invention to provide a training system for an artificial intelligence entity that can improve the classification of medical images, in particular of blood cell images, with artificial neural networks without the need of meta-data.

The purpose of this invention will be achieved through a device with the features disclosed in claim 1 and a method with the features detailed in claim 15. Preferred embodiments of the invention with advantageous features are given in the dependent claims.

A first aspect of the invention provides a training system for training an artificial neural network, in particular for medical applications, comprising an image-processing device, which is configured to receive a set of acquired images, an artificial intelligence module, configured to implement an artificial neural network, and a controller device, configured to train the artificial neural network implemented by the artificial intelligence module, wherein the image-processing device is further configured to generate modified images based on a modification of the acquired images, wherein each of the modified images generated by the image-processing device contains less texture information than the respective acquired image from which they stem, and wherein the controller device is configured to receive a set of training images comprising at least part of the modified images and at least part of the acquired images and train an artificial neural network which, based on a classification scheme, is configured to output a probability distribution for each training image according to the classification scheme.

The set of acquired images broadly describes any visual information that can be used for further processing and analysis in medical applications. In the context of blood cell classification, the set of acquired images consist of artificially-stained blood samples (or: stained blood smears), using any of the staining methods used in blood cell classification, e.g. when performed by microscope examination.

Image features can be split into texture (or: textural) features and structure (or: structural) features. Here and in the following, structural features of an image will be understood as information contained in the image or properties of the image that have an intrinsic geometric meaning (e.g. size of a cell, shape of the cell, relative size of the cell nucleus with respect to the total cell size). In contrast, texture features of an image will be understood as information contained in the image or properties of the image that has to do with the cell color (before and/or after staining) or the granularity of the cell. Sometimes texture features are also denoted as local features, while structural features are described as global features.

A modified image with less texture features than the original acquired image it originated from should be understood as an image in which the modification is aimed at an enhancement of geometric features at the expense of the texture features. Modifications of this kind will be mentioned in the following when describing the different preferred embodiments contained in the dependent claims and the figures.

The image-processing device is broadly understood as any entity capable of performing modifications on a set of images. The image-processing device may therefore contain, at least, a central processing unit, CPU, and/or at least one graphics processing unit, GPU, and/or at least one field-programmable gate array, FPGA, and/or at least one application-specific integrated circuit, ASIC and/or any combination of the foregoing. It may further comprise a working memory operatively connected to the at least one CPU and/or a non-transitory memory operatively connected to the at least one CPU and/or the working memory. The image-processing device may execute a software, an app or an algorithm with different capabilities for image editing. It may be implemented partially and/or completely in a local apparatus and/or partially and/or completely in a remote system such as by a cloud computing platform.

Whenever herein an artificial intelligence module is mentioned, it shall be understood as a computerized entity able to implement different data analysis methods broadly described under the terms artificial intelligence, machine learning, deep learning or computer learning.

The controller device is to be broadly understood as an entity able to process data. The controller device can be realized as any device containing or consisting of, at least, a central processing unit, CPU, and/or at least one graphics processing unit, GPU, and/or at least one field-programmable gate array, FPGA, and/or at least one application-specific integrated circuit, ASIC and/or any combination of the foregoing. It may further comprise a working memory operatively connected to the at least one CPU and/or a non-transitory memory operatively connected to the at least one CPU and/or the working memory. The controller device may execute a software, an app or an algorithm with different capabilities for data processing. It may be implemented partially and/or completely in a local apparatus and/or partially and/or completely in a remote system such as by a cloud computing platform.

A second aspect of the present invention provides a training method for training an artificial neural network, preferably the training system of the first aspect, comprising the following steps: (a) acquiring a set of images; (b) generating a set of images based on a modification of the acquired images, wherein each of the modified images contains less texture information than the respective acquired image from which they stem; (c) initializing an artificial neural network; and (d) training the artificial neural network with training images comprising at least part of the modified images and at least part of the acquired images.

In particular, the method according to the second aspect of the invention may be carried out with the training device according to the first aspect of the invention. The features and advantages disclosed herein in connection with the image-forming device are therefore also disclosed for the method, and vice versa.

According to a third aspect, the invention provides a computer program product comprising executable program code configured to, when executed, perform the method according to the second aspect of the present invention.

According to a fourth aspect, the invention provides a non-transient computer-readable data storage medium comprising executable program code configured to, when executed, perform the method according to the second aspect of the present invention.

The non-transient computer-readable data storage medium may comprise, or consist of, any type of computer memory, in particular semiconductor memory such as a solid-state memory. The data storage medium may also comprise, or consist of, a CD, a DVD, a Blu-Ray-Disc, an USB memory stick or the like.

According to a fifth aspect, the invention provides a data stream comprising, or configured to generate, executable program code configured to, when executed, perform the method according to the second aspect of the present invention.

One of the main ideas underlying the present invention is to provide a training system, configured to train an artificial intelligence neural network for the classification of medical image data, in which the training images contain modified images. The modifications that generate the modified images are aimed at reducing textural information with a corresponding increase of structural information. This modified set of images is added to the initially acquired medical images to generate an enlarged dataset, with which the artificial neural network is then trained on a classification scheme.

The device as described above affords a simple implementation of a method to train an artificial neural network for the classification of medical images. One step consists in acquiring the medical images, which in most cases have previously undergone a staining process. A certain subset of the acquired images are modified, thereby generating new images. The acquired image dataset together with the modified images are used to build an enlarged (or: augmented) image dataset. The artificial neural network is initialized and then trained with the images of the augmented image dataset according to a classification scheme that depends on the specific medical application. A probability distribution for each image according to the classification scheme is generated.

An advantage of the present invention resides in that it balances a deficit in the training of some artificial neural network, in particular convolutional neural networks, which tend to outweigh textural features over structural features. In other words, the recognition and extraction of textural features from images happens to be significantly easier for a neural network than the recognition and extraction of structural features. In the present invention, this bias of certain artificial neural networks is (at least partially) countered, or compensated for, by the addition of a modified image dataset, where textural features have been substantially removed. The modifications deplete the initial images from texture features, thus enhancing the relative weight of the corresponding structural features. This leads to a training dataset where both textural and structural features are trained into the artificial neural network, thereby improving the performance of the artificial neural network as a classification tool.

Another advantage of the present invention is that the trained artificial neural network will perform much better in classifications where some of the classes have strong structural features. For instance, monocytes and activated lymphocytes can be distinguished from other leucocytes by their (large) size much better than through their textural features. Therefore, the classification achieved with the training system of the invention for such structurally distinct classes will be rather robust.

A further advantage of the present invention is that no meta-data, i.e. data external to the images, is needed for the classification. No extracted information from the images (e.g. size of the cells, excentricity of the cell nucleus or presence of vacuoles) nor patient-specific information (e.g. gender, age or medical history) are required. All the information is contained in the acquired images and in the associated modified images.

Advantageous embodiments and further developments follow from the dependent claims as well as from the description of the different preferred embodiments illustrated in the accompanying figures.

According to some of the embodiments, refinements, or variants of embodiments, the artificial neural network is configured to classify an image based on the probability distribution associated to the image. In other words, the artificial neural network can post-process the probability distribution associated to an image and assign a class label to the image.

According to some of the embodiments, refinements, or variants of embodiments, the image-processing device further comprises a color-removal module, which is configured to perform a modification of an image in a first selection by removing the color of the image. The color-removal module is configured to receive an acquired image and generate a modified image where the color has been removed and converted, e.g. through a standard linear conversion, into a grey-scaled image. This is one of the most fundamental modifications that can be performed to remove textural features from the acquired images.

According to some of the embodiments, refinements, or variants of embodiments, the image-processing device further comprises an edge-detection module, which is configured to perform a modification of an image in a second selection by detecting the edges of the image. Another fundamental modification that can be performed on the acquired images is a so-called edge detection, e.g. with a Canny algorithm. The edge-detection module is configured to receive an acquired image and produce a black and white modified image, where only the contours (defined by a certain contrast threshold of the algorithm) are retained. This modification therefore removes color and other texture information.

According to some of the embodiments, refinements, or variants of embodiments, the image-processing device further comprises a segmentation module, which is configured to perform a modification of an image in a third selection by segmenting parts of the image. The segmentation module is configured to identify different cell constituents (e.g. cell nucleus, cell plasma and vacuoles) and erase their internal texture, e.g. by replacing all the pixels corresponding to a cell constituent by a constant color. The resulting modified image therefore contains the different cell constituents identified by different colors.

According to some of the embodiments, refinements, or variants of embodiments, the image-processing device further comprises a filtering module, which is configured to perform a modification of an image in a fourth selection by smearing and blurring the image. This selection is based on a filtering technique (e.g. implemented through a vector median filter or a Gaussian filter), where local patterns are smeared over neighboring pixels, resulting in a smoothed or blurred image and a corresponding removal of texture features.

The image modifications operated by the different modules of the image-processing device are not exclusive and can be combined in different ways. For instance, vector median filters and Gaussian filters are normally employed in the initial stages of edge detection.

According to some of the embodiments, refinements, or variants of embodiments, the image-processing device further comprises a transformation module, which is configured to further modify a modified image by applying a set of transformations, comprising rotations and/or translations and/or modification of the brightness and/or modification of the contrast and/or modification of the color intensity. The above set of transformations increases the robustness of the structural features, especially if there are added on top of the modifications spelled in the previous paragraphs. For instance, by rotating a modified image with edge detection one makes sure that the space orientation of the relevant object (e.g. a monocyte) is no longer significant, only the shape of it. Therefore, it is possible, and in many applications even desirable, to combine a set of modifications and then a set of transformations. The combinatorial possibilities are unlimited and depend, among other things, on the specific medical application. One could, for instance, perform two rotations of the monocyte mentioned above with different angles and keep both generated images as part of the enlarged dataset for training. This augmentation of the image data therefore has two main advantages: first, it makes the training robust against the transformations; and second, it increases the number of training images without requiring extra acquired images.

According to some of the embodiments, refinements, or variants of embodiments, the controller device further comprises a selector module, which is configured to select the modified images from the first selection and/or the second selection and/or the third selection and/or the fourth selection and the transformed images and build training data therewith.

As already mentioned in the foregoing, the modified data can be obtained from the different selection mechanisms, either in isolation or in combination. The selector module may do the selection a posteriori, i.e. based on data already modified by the image-processing unit or, alternatively, can send a customized command to the image-processing unit for it to generate a particular composition of the modified data. The first option speeds up the augmentation process but increases the size of the training data, while the second option has the advantage that the training data is generated on-the-fly. The proportion of the different modifications and transformation in this composition can be predetermined or randomly generated. Likewise, the acquired images from which modified images will be generated using a certain selection can be randomly chosen or predetermined.

According to some of the embodiments, refinements, or variants of embodiments, the proportion of modified images corresponding to the first selection, the second selection, the third selection and the fourth selection and the transformed images in the training data is updated for each training epoch. In other words, whether an acquired image is modified or not and with which selection it is modified, is determined at the beginning of every epoch for the whole set of acquired images. This process is repeated at the beginning of each epoch, such that e.g. one acquired image that got modified with the third selection in a first epoch, can be unmodified in a second epoch, modified with the second selection in a third epoch, and again modified with the third selection in a fourth epoch. Thus, over the hundred or more epochs of a full training, very different configurations of the training data are generated.

According to some of the embodiments, refinements, or variants of embodiments, the artificial neural network comprises a convolutional neural network. While the principle upon which the invention is based has a broad range of application for any artificial neural network, convolutional neural networks are very commonly used for medical image analysis. Therefore, the principles of the invention are especially beneficial when working with convolutional neural networks.

According to some of the embodiments, refinements, or variants of embodiments, the acquired images comprise or consist of images of blood samples. The classification scheme of the artificial neural network can be used in these cases for the early detection and diagnosis or abnormalities that can be detected in the blood.

According to some of the embodiments, refinements, or variants of embodiments, the classes in the classification scheme correspond at least to types of leucocytes. The classification scheme of the artificial neural network can be used in these cases for the early detection and diagnosis or abnormalities that affect the immune system.

According to some of the embodiments, refinements, or variants of embodiments, the classes in the classification scheme correspond to types of leucocytes and at least one additional class corresponding to anomalous leucocytes. Leucocytes can display morphological differences with respect to the canonical features which describe each of their classes, either because of issues related to the acquired images (angle of exposure of the cell, excessive staining), because certain types of cells are morphologically different depending on their maturation stage, or simply because they are abnormal as a manifestation of a disease. An improved classification such as the one proposed in the invention can not only help classify leucocytes better, but at the same time it is more sensitive to abnormal morphologies, which are strong indicators of diseases.

According to some of the embodiments, refinements, or variants of embodiments, the training images contain between 10% and 30%, preferably 20%, of modified images. The enlarged or augmented dataset that is used as training images for the artificial neural network is meant to reinforce structural features, which can be more easily recognized and extracted by the artificial neural network. However, the training images ought to contain also acquired data, such that texture features and other information are not altogether lost. Ideally, one should find the minimal amount of modified data needed to enhanced structural features to an acceptable level. According to some configurations investigated by the inventors, a 10% to 30% increase of the acquired data is, in this respect, a sensible amount. This proportion is however expected to vary depending on the application and on the training methodology adopted. In configurations where structural features are hard to be extracted, one can expect that a larger amount of modified images will be needed for the training of these features. Furthermore, one can imagine step-based trainings in the spirit of transfer learning, where a first training is performed without modified images, and a second training is performed including modified images. In the second training, a large proportion of modified images is to be expected in order to balance out the already learned texture features during the first training.

Although here, in the foregoing and also in the following, some functions are described as being performed by modules, it shall be understood that this does not necessarily mean that such modules are provided as entities separate from one another. In cases where one or more modules are provided as software, the modules may be implemented by program code sections or program code snippets, which may be distinct from one another but which, may also be interwoven or integrated into one another.

Similarly, in cases where one or more modules are provided as hardware, the functions of one or more modules may be provided by one and the same hardware component, or the functions of several modules may be distributed over several hardware components, which need not necessarily correspond to the modules. Thus, any apparatus, system, method and so on which exhibits all of the features and functions ascribed to a specific module shall be understood to comprise, or implement, said module. In particular, it is a possibility that all modules are implemented by program code executed by the computing device, for example a server or a cloud computing platform.

The above embodiments and implementations can be combined with each other as desired, as far as this is reasonable.

Further scope of the applicability of the present method and apparatus will become apparent from the following figures, detailed description and claims. However, it should be understood that the detailed description and specific examples, while indicating preferred embodiments of the invention, are given by way of illustration only, since various changes and modifications within the spirit and scope of the invention will become apparent to those skilled in the art.

Aspects of the present disclosure will be better understood with reference to the following figures. The components in the drawings are not necessarily to scale, emphasis being placed instead upon clearly illustrating the principles of the present disclosure. Parts in the different figures that correspond to the same elements have been tagged with the same reference numerals in the figures.
FIG. 1 is a schematic depiction of a training system according to an embodiment of the present invention;
FIG. 2 is a schematic depiction of two possible configurations of selections in the build-up of the training data according to an embodiment of the present invention;
FIG. 3 is a block diagram showing an exemplary embodiment of the training method;
FIG. 4 is a schematic illustration of an edge detection procedure applied to a leucocyte using the Canny algorithm according to an embodiment of the present invention;
FIG. 5 is a schematic illustration of a segmentation procedure applied to a leucocyte according to an embodiment of the present invention;
FIG. 6 shows a confusion matrix corresponding to the probability distribution outputted by the artificial intelligence module according to an embodiment of the present invention;
FIG. 7 is a schematic block diagram illustrating a computer program product according to an embodiment of the third aspect of the present invention; and
FIG. 8 is a schematic block diagram illustrating a non-transitory computer-readable data storage medium according to an embodiment of the fourth aspect of the present invention.

The figures might not be to scale and certain components can be shown in generalized or schematic form in the interest of clarity and conciseness. In some instances, well-known structures and devices are shown in block diagram form in order to avoid obscuring the concepts of the present invention. Likewise, the numeration of the steps in the methods are meant to ease their description. They do not necessarily imply a certain ordering of the steps. In particular, several steps may be performed concurrently.

The detailed description includes specific details for the purpose of providing a thorough understanding of the present invention. However, it will be apparent to those skilled in the art that the present invention may be practised without these specific details.

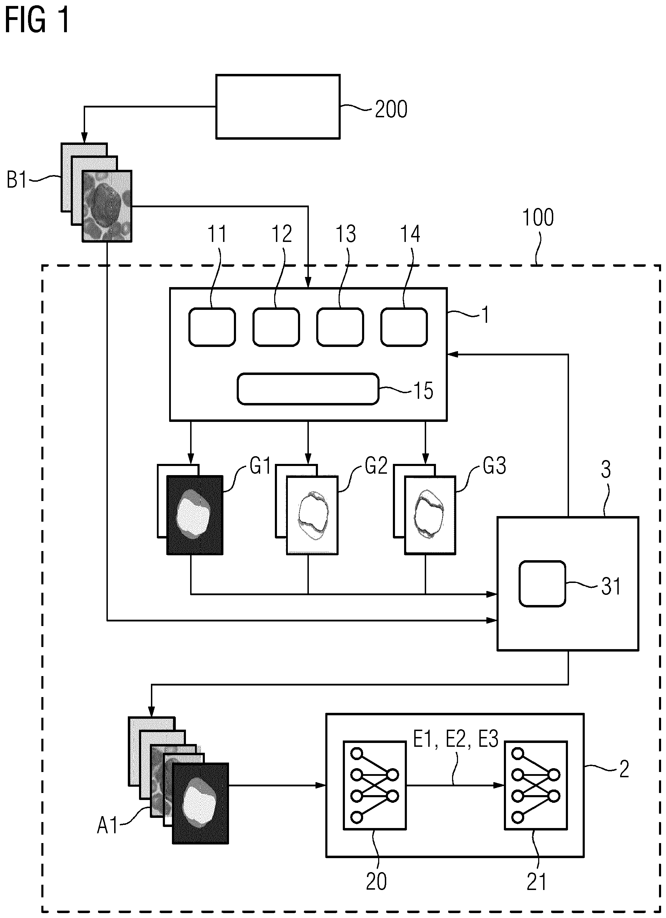

FIG. 1 is a schematic depiction of a training system 100 according to an embodiment of the present invention. The figure also shows an image-storage unit 200, e.g. a picture archiving and communication system (PACS), where a set of medical images B1 can be stored and retrieved for analysis.

The medical images B1 can be any visual source of information susceptible to be used for further processing and analysis in medical applications. In the context of blood cell classification, such medical images B1 may consist of stained blood smears, generated using any of the standard staining methods.

The training system 100 comprises an image-processing device 1, an artificial intelligence module 2 and a controller device 3. All these elements are connected among each other either wire-bound or wireless.

The image-processing device 1 is broadly understood as any entity capable of performing modifications on a set of images. In FIG. 1, the image-processing device 1 is configured to retrieve medical images B1 (or: acquired images) from the PACS 200 for further editing and eventually generate a set or set of modified data G1, G2, G3. The image-processing device 1 may therefore contain, at least, a central processing unit, CPU, and/or at least one graphics processing unit, GPU, and/or at least one field-programmable gate array, FPGA, and/or at least one application-specific integrated circuit, ASIC and/or any combination of the foregoing. For simplicity, these different elements are not shown in the figure. It may further comprise a working memory operatively connected to the at least one CPU and/or a non-transitory memory operatively connected to the at least one CPU and/or the working memory. For the same reason as before, these elements are not shown in the figure. The image-processing device 1 is configured to execute one or more software, apps or algorithms with different capabilities for image editing. It may be implemented partially and/or completely in a local apparatus, as shown in the figure, and/or partially and/or completely in a remote system such as by a cloud-computing platform.

In the embodiment of FIG. 1, the image-processing device 1 further contains a number of modules 11, 12, 13, 14, 15, for image editing. As an example, FIG. 1 shows a color-removal module 11, an edge-detection module 12, a segmentation module 13, a filtering module 14 and a transformation module 15.

Given an image from the acquired images B1, the color-removal module 11 is configured to generate a modified image thereof, in which the color is removed and the image is converted to a grey-scale one, e.g. through a standard linear conversion, with the luminance given by the relation
Y=0.3*R+0.59*G+0.11*B, Y being the luminance, and R, G and B the red, green and blue intensity levels, respectively.

The edge-detection module 12 is configured to generate a black and white contour image of a given image belonging to the acquired images B1, e.g. with the so-called Canny algorithm, where only the contours defined by a certain contrast threshold of the algorithm are retained.

The segmentation module 13 is configured to identify different cell constituents (e.g. cell nucleus, cell plasma and vacuoles) from an image and erase their internal texture, e.g. by replacing all the pixels corresponding to a cell constituent by a constant color. The resulting modified image therefore contains the different cell constituents identified by different colors.

The filtering module 14 is configured to perform a modification of a given acquired image B1 based on a filtering technique or process (e.g. implemented through a vector median filter or a Gaussian filter). This filtering process smears local patterns over neighboring pixels, creating a smoothed or blurred image.

The image modifications performed by the different modules 11, 12, 13, 14 of the image-processing device 1 can be further combined to generate a set or different sets of modified images G1, G2, G3. The main goal of the modifications introduced is to diminish or reduce or eliminate textural features from the acquired images B1. These features happen to be recognized and extracted by artificial neural networks 20 more easily than structural features, which leads to an over-weighting of textural features in image recognition. One way to compensate for this bias is to generate training images A1 by adding to the acquired images B1 sets of modified images G1, G2, G3 depleted from textural features, or at least with reduced textural features, and increase the presence of structural features (i.e. geometric features).

The transformation module 15 is configured to further modify the generated data by applying a set of transformations, comprising rotations and/or translations and/or modification of the brightness and/or modification of the contrast and/or modification of the color intensity. The above set of transformations increases or reinforces the robustness of the structural features, especially if these transformations are added on top of the modifications performed by the other modules 11, 12, 13, 14 of the image-processing device 1.

The artificial intelligence module 2 is any computerized entity able to implement different data analysis methods broadly described under the terms artificial intelligence, machine learning, deep learning or computer learning. In the embodiment of FIG. 1, the artificial intelligence module 2 comprises an artificial neural network 20, 21, preferably a convolutional neural network, which is trained with the training images A1, consisting of the acquired images B1 and a set of modified data G1, G2, G3, according to a predetermined classification scheme, e.g. leucocyte classification. For training purposes a standard architecture, e.g. RenGen18 is used, with a training algorithm based on gradient descent with learning rate scheduling, momentum and gradient decay. The training data A1 can be divided in batches and fed into the untrained artificial neural network 20 for a number of epochs E1, E2, E3, after which a trained artificial neural network 21 is available, whose output is a probability distribution for each input image according to the classification scheme and the assignment of an identification label to each image.

The controller device 3 is an entity able to process and manage data. It contains or consists of, at least, a central processing unit, CPU, and/or at least one graphics processing unit, GPU, and/or at least one field-programmable gate array, FPGA, and/or at least one application-specific integrated circuit, ASIC and/or any combination of the foregoing. For simplicity, these elements are not shown in FIG. 1. It may further comprise a working memory operatively connected to the at least one CPU and/or a non-transitory memory operatively connected to the at least one CPU and/or the working memory, likewise not shown in the figure. The controller device 3 may execute software, apps or algorithms with different capabilities for data processing and management. It may be implemented partially and/or completely in a local apparatus, as shown in FIG. 1, and/or partially and/or completely in a remote system such as by a cloud computing platform.

In particular, the image-processing device 1 and the controller device 3 may be implemented by the same device or devices.

The controller device 3 comprises a selector module 31, which is configured to select which modification of the acquired images B1 has to be added to the training images A1 by activating the different modules 11, 12, 13, 14, 15 of the image-processing device 1. This selection is based both on the proportion of modified images G1, G2, G3 that the training images A1 should contain and/or on the nature of the transformations and modifications to be used in the generation of the modified images G1, G2, G3. For instance, in some preferred embodiments the modified images G1, G2, G3 comprise between 10% and 30%, preferably 20%, of the training images A1. From this percentage, e.g. 50% of the acquired images B1 should be modified by the color-removal module, and the remaining 50% by the filtering module. The actual images to be modified can be randomly sampled from the pool of acquired images B1 or preselected. In preferred embodiments, these proportions can be fixed independently for each epoch E1, E2, E3 of the training. Further implementations and generalizations are possible, e.g. applying a different selection of parameters for each batch within a training epoch E1, E2, E3, or combining random selections of images with predetermined ones.

According to the selections of the selection module 31, the controller device 3 may either extract already modified image data from the image-processing device 1 or, preferably, instruct the image-processing device 1 to generate the modified data G1, G2, G3 on demand.

The controller device 3 is further configured to prepare the training images A1 to be fed to the artificial intelligence module 2 for the training of the (untrained) artificial neural network 20 at each training epoch E1, E2, E3.

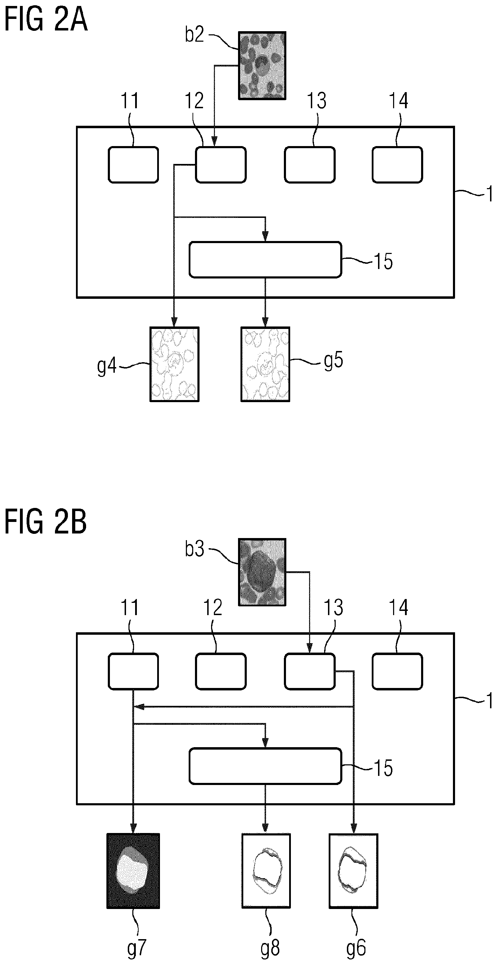

FIG. 2 is a schematic depiction of two possible configurations of selections (or: augmentations) in the build-up of the training data A1 according to an embodiment of the present invention.

FIG. 2A shows the image-processing unit 1 of FIG. 1 with a color-removal module 11, an edge-detection module 12, a segmentation module 13, a filtering module and a transformation module 15. The selector module 31 generates a selection that determines how the acquired images are to be processed and modified. For instance, the selection may specify that 15% of the acquired images B1 should be modified, 30% of which through the color-removal module and the remaining 60% of which with the edge-detection module. The remaining 10% of the modified images G1, G2, G3 are then generated from rotations of the image modified through edge detection.

Specifically, FIG. 2A shows how the selection described above could affect a single image b2. The image b2 in FIG. 2A undergoes an edge-detection modification operated by the edge-detection module 12, which produces a generated image g4. This same image is further modified by a 180-degree rotation operated by the transformation module 15, resulting in a generated image g5.

The controller device 3 can be configured to collect the generated images b4 and b5 and add them to the acquired image b2 to form an augmented image dataset of training images A1, which can be input into the artificial intelligence module 2 and used for training the artificial neural network 20. Alternatively, the controller device 3 can be configured to collect only g5 as part of the generated image dataset and add it to b2 to build a dataset of training images A1. It is even possible that only b4 and/or b5 are included in the set of training images A1. In this case, the acquired images B1 within the training images A1 are therefore chosen from the non-modified ones. This final step may depend on the configuration chosen by the selection module 31, which sets, among other things, the proportion of modified images g4, g5 in the training images A1.

FIG. 2B shows another possible path to augmentation of the acquired medical images B1. In this case, an acquired image b3 is processed first by the segmentation module 13. The resulting modified image g6 shows the characteristic color separation between nucleus and plasma of the cell. The segmented image g6 is further processed by the color-removal module 11, resulting in the generated image g7. In turn, g7 is further processed via a 180-degree rotation operated by the transformation module 15, resulting in the generated image g8.

The so modified images g6, g7 and g8 provide an example of how the image-processing unit 1 can augment the data, in this case from a single image b3. As before, depending on the settings of the selection module 31, the controller device 3 will collect all the generated images g6, g7, g8 or only a subset thereof. Likewise, b3 might be kept as part of the training images A1 or dropped from it.

As mentioned in the foregoing, adding modifications operated by the transformation module 15 to the training images A1 tend to the reinforce the role of structural features. In some embodiments it is therefore preferred to keep data modified by the color-removal module 11 and/or the edge-detection module 12 and/or the segmentation module 13 and/or the filtering module 14 together with further modifications of this same data performed by the transformation module 15.

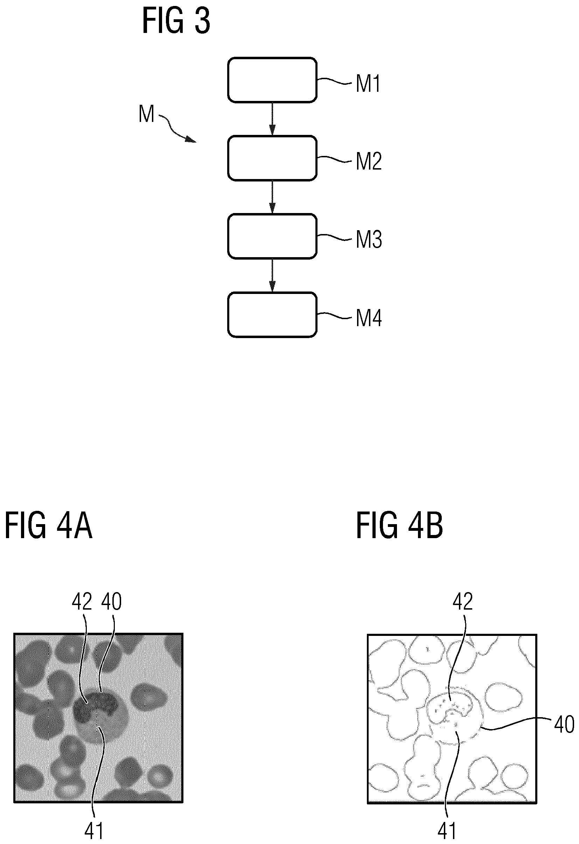

FIG. 3 is a block diagram showing an exemplary embodiment of the training method M, to be applied preferentially with a training system 100 as described in FIG. 1 and FIG. 2. One step M1 consists in acquiring a set of medical image data B1, b2, b3, which in the preferred embodiment of white blood cell classification in most cases has previously undergone a staining process. In another step M2, a certain subset of the acquired images B1, b2, b3 is modified, thereby generating new images G1, G2, G3, g4, g5, g6, g7, g8, wherein each of the modified images G1, G2, G3, g4, g5, g6, g7, g8 contains less texture information than the respective acquired image B1, b2, b3 from which they were generated. How, from this subset of acquired data B1, b2, b3, the modified images G1, G2, G3, g4, g5, g6, g7, g8 can be generated has been discussed above in the description of FIG. 2. At least part of the acquired image dataset B1, b2, b3 together with the modified images G1, G2, G3, g4, g5, g6, g7, g8 are used to build an enlarged dataset A1. In another step M3, the artificial neural network 20 is initialized and then trained, in a step M4, using the training images A1 according to a classification scheme that depends on the specific medical application.

Once the training process M is completed, e.g. with a classification scheme for the identification of the different types of leucocytes, the artificial neural network 21 can be validated and then used for the classification of leucocytes from new smear blood images. The probability distribution that comes out of the artificial neural network 21 for each input image can be post-processed in order to assign an identification label, e.g. type of leucocyte, to each image.

FIG. 4 is a schematic illustration of an edge detection procedure applied to a leucocyte using the Canny algorithm according to a variant of an embodiment of the present invention. In particular, it may be the edge detection procedure in FIG. 2A, which generates the modified image g4 from the acquired image b2.

FIG. 4A is an image corresponding to a stained blood sample. In the central part of the image there is a leucocyte 40, from which the nucleus 42 and the plasma 41 can be distinguished. FIG. 4B shows a modified black and white image after an edge-detection procedure was carried using the Canny algorithm. The algorithm fares better the higher the contrast is between the different regions to be delimited. The contrast between the nucleus 42 and the plasma 41 is marked, while the contrast between the leucocyte 40 and the background of FIG. 4B is less marked. Accordingly, as can be seen from the figure, the algorithm is able to neatly distinguish the contour of the nucleus 42. The contour of the leucocyte 40 is however harder to detect and the contour is only partially recognized. FIG. 4b is an example of a modified image g4 (see FIG. 2A) generated by the edge-detection module 12. With edge-detection, both color and additional textural features are removed, thus reinforcing structural features.

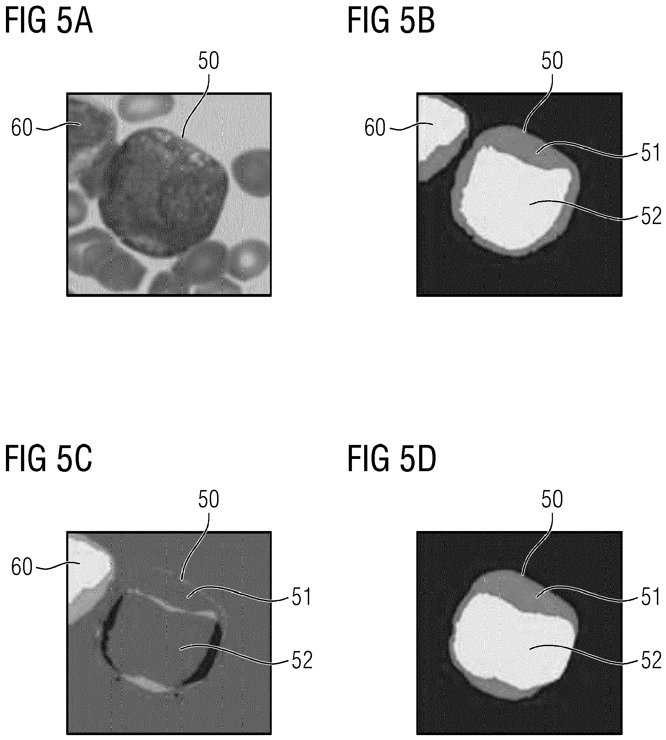

FIG. 5 is a schematic illustration of a segmentation procedure applied to a leucocyte according to a variant of an embodiment of the present invention. In particular, it may be the segmentation procedure in FIG. 2B, which generates the modified image g6 from the acquired image b3.

FIG. 5A is an acquired image corresponding to a stained blood sample. In the central part of the image there is a first leucocyte 50, from which at naked eye no internal structure can be seen. On the left-hand upper corner of the figure there is a second leucocyte 60, which is only partially shown.

FIG. 5b shows an image modification of FIG. 5A through a segmentation procedure carried by a biological lab expert. Coloring has been applied to identify the nucleus 52 and the plasma 51 and the background structures present in FIG. 5A have been filtered out. The segmentation procedure works in such a way that different structures get uniform coloring, which results in a loss of textural information. In FIG. 5B the nucleus 52 and the plasma 51 are neatly distinguishable, and also the second leucocyte 60 is still present.

FIG. 5D shows, in contrast and for comparison, an image modification of FIG. 5A through a segmentation procedure, this time carried by an artificial neural network, not necessarily the one of the invention. The segmentation algorithm applies coloring to the nucleus 52 and the plasma 51 and filters out the background structures present in FIG. 5A, including the second leucocyte 60. FIG. 5D is an example of a modified image g6 (see FIG. 2D) that could be used as part of the training data A1.

FIG. 5C shows a comparison between the segmentation procedures applied to FIG. 5A carried by an expert, leading to FIG. 5B, and by an artificial neural network (not necessarily the one of the invention), leading to FIG. 5D. From FIG. 5C, one identifies more easily that the identifications of plasma 51 and nucleus 52 in FIG. 5B and FIG. 5D are in very good agreement.

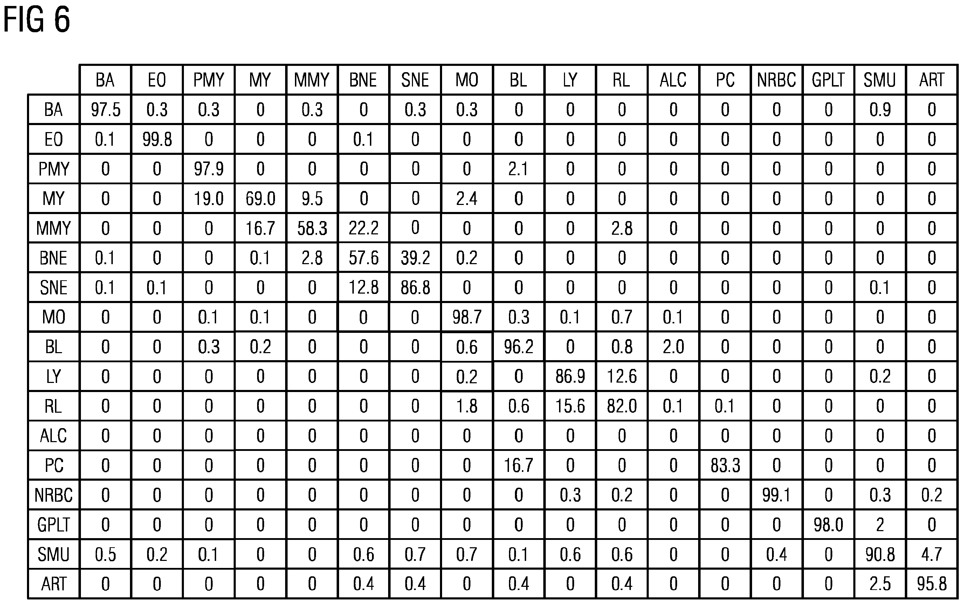

FIG. 6 shows a confusion matrix corresponding to the probability distribution outputted by the artificial intelligence neural network 21 according to a variant of an embodiment of the present invention. The classification scheme is shown by the labels in the first column and row and corresponds to a classification scheme for leucocytes with 17 different classes, namely basophils (BA), eosinophils (EO), promyelocytes (PMY), myelocytes (MY), metamyelocytes (MMY), band neutrophils (BNE), segmented neutrophils (SNE), monocytes (MO), blasts (BL), lymphocytes (LY), reactive lymphocytes (RL), abnormal lymphocytes (ALC), plasma cells (PC), nucleated red blood cells (NRBC), giant platelets (GPLT), smudge (SMU) and artefacts (ART). The classification scheme therefore includes the 5 types of leucocytes (basophils, eosinophils, neutrophils, monocytes and lymphocytes) but also cell parts that are not leucocytes, namely, the last 6 classes. This is important in order to test the accuracy of the artificial neural network 21 as a classifier. Furthermore, neutrophils are classified according to different stages of their maturation (classes PMY to SNE).

The confusion matrix of FIG. 6 shows the outcome of the trained artificial neural network 21 after a validation step. From the matrix it is clear that the artificial neural network 21 identifies successfully (with a probability of above 99%) both EO and NRBC. EO are only misidentified with BA and BNE, but the misidentification amounts to only 0.1% for each class. NRBC can be misidentified with SMU, ART or lymphocytes (LY and RY), again with very small probability. In the data used there happen to be no ALC, which explains why the corresponding row is empty.

For the purpose of the invention it is important to highlight monocytes, blasts, promyelocytes and lymphocytes. It has been already mentioned in the foregoing that one of the most advantageous aspects of the invention is related to classes that are structurally different from the others. In particular, monocytes, blasts and promyelocytes are substantially bigger than other leucocytes. Using augmentation in training data to emphasize structural features while removing textural features according to the present invention results in an artificial neural network 21 that distinguishes these classes neatly. The identification rates for monocytes, blasts and promyelocyte are above 98%, 96% and 97%, respectively, which is a strong indication that structural features, and in particular the cell size, have been learned by the artificial neural network 21.

The misidentification between monocytes and lymphocytes is also reduced below the 1% level. The inventors have verified that with an augmentation using only color removal, i.e. by adding modified grey-scaled images to the training data, the misidentification between monocytes and lymphocytes gets reduced substantially.

The entries corresponding to monocytes, blasts, promyelocytes and lymphocytes in the confusion matrix in FIG. 6 therefore show that structural features are indeed contained in the artificial neural network 21, and that they can be used to improve the classification of leucocytes.

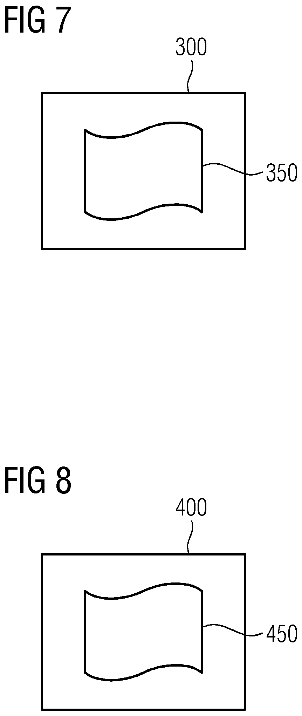

FIG. 7 shows a schematic block diagram illustrating a computer program product 300 according to an embodiment of the third aspect of the present invention. The computer program product 300 comprises executable program code 350 configured to, when executed, perform the method according to any embodiment of the second aspect of the present invention, in particular as has been described with respect to the preceding figures.

FIG. 8 shows a schematic block diagram illustrating a non-transitory computer-readable data storage medium 400 according to an embodiment of the fourth aspect of the present invention. The data storage medium 400 comprises executable program code 450 configured to, when executed, perform the method according to any embodiment of the second aspect of the present invention, in particular as has been described with respect to the preceding figures.

The non-transient computer-readable data storage medium may comprise, or consist of, any type of computer memory, in particular semiconductor memory such as a solid-state memory. The data storage medium may also comprise, or consist of, a CD, a DVD, a Blu-Ray-Disc, an USB memory stick or the like.

The previous description of the disclosed embodiments are merely examples of possible implementations, which are provided to enable any person skilled in the art to make or use the present invention. Various variations and modifications of these embodiments will be readily apparent to those skilled in the art, and the generic principles defined herein may be applied to other embodiments without departing from the spirit or scope of the present disclosure. Thus, the present invention is not intended to be limited to the embodiments shown herein but it is to be accorded the widest scope consistent with the principles and novel features disclosed herein. Therefore, the present invention is not to be limited except in accordance with the following claims.

### List of Reference Signs

- 1: image-processing device
- 2: artificial intelligence module
- 3: controller device
- 11: color-removal module
- 12: edge-detection module
- 13: segmentation module
- 14: filtering module
- 15: transformation module
- 20: artificial neural network
- 21: artificial neural network
- 31: selector module
- 40: leucocyte
- 41: plasma
- 42: nucleus
- 50: first leucocyte
- 51: plasma
- 52: nucleus
- 60: second leucocyte
- 100: training system
- 200: image storage unit
- 300: computer program product
- 350: executable program code
- 400: non-transitory computer-readable data storage medium
- 450: executable program code

- A1: training images
- B1: acquired images
- b2: acquired image
- b3: acquired image
- G1...G3: modified images
- g4...g8: modified image

- M1...M4: method steps

## Claims

1. A training system (100) for training an artificial neural network (20), in particular for medical applications, comprising:
an image-processing device (1), which is configured to receive a set of acquired images (B1, b2, b3);
an artificial intelligence module (2), configured to implement an artificial neural network (20, 21); and
a controller device (3), configured to train the artificial neural network (20) implemented by the artificial intelligence module (2);
wherein the image-processing device (1) is further configured to generate modified images (G1, G2, G3, g4, g5, g6, g7, g8) based on a modification of the acquired images (B1, b2, b3);
wherein each of the modified images (G1, G2, G3, g4, g5, g6, g7, g8) generated by the image-processing device (1) contains less texture information than the respective acquired image (B1, b2, b3) from which they stem; and
wherein the controller device (3) is configured to receive a set of training images (A1) comprising at least part of the modified images (G1, G2, G3, g4, g5, g6, g7, g8) and at least part of the acquired images (B1, b2, b3) and train an artificial neural network (20) which, based on a classification scheme, is configured to output a probability distribution for each training image (A1) according to the classification scheme.

2. The training system (100) according to claim 1, wherein the artificial neural network (20, 21) is configured to classify an image based on the probability distribution associated to the image.

3. The training system (100) according to claims 1 and 2, wherein the image-processing device (1) further comprises a color-removal module (11), which is configured to perform a modification of an image in a first selection by removing the color of the image.

4. The training system (100) according to claims 1 and 2, wherein the image-processing device (1) further comprises an edge-detection module (12), which is configured to perform a modification of an image in a second selection by detecting the edges of the image.

5. The training system (100) according to claims 1 and 2, wherein the image-processing device (1) further comprises a segmentation module (13), which is configured to perform a modification of an image in a third selection by segmenting parts of the image.

6. The training system (100) according to claims 1 and 2, wherein the image-processing device (1) further comprises a filtering module (14), which is configured to perform a modification of an image in a fourth selection by smearing and blurring the image.

7. The training system (100) according to claims 1 to 6, wherein the image-processing device (1) further comprises a transformation module (15), which is configured to further modify a modified image (G1, G2, G3, g4, g5, g6, g7, g8) by applying a set of transformations, comprising rotations and/or translations and/or modification of the brightness and/or modification of the contrast and/or modification of the color intensity.

8. The training system (100) according to claims 3 to 7, wherein the controller device (3) further comprises a selector module (31), which is configured to select the modified images (G1, G2, G3, g4, g5, g6, g7, g8) from the first selection and/or the second selection and/or the third selection and/or the fourth selection and the transformed images and build training data (A1) therewith.

9. The training system (100) according to claims 8, wherein the proportion of modified images (G1, G2, G3, g4, g5, g6, g7, g8) corresponding to the first selection, the second selection, the third selection and the fourth selection and the transformed images in the training data (A1) is updated for each training epoch (E1, E2, E3).

10. The training system (100) according to claims 1 to 9, wherein the artificial neural network (20, 21) comprises a convolutional neural network.

11. The training system (100) according to claims 1 to 10, wherein the acquired images (B1) comprise or consist of images of blood samples.

12. The training system (100) according to claims 1 to 11, wherein the classes in the classification scheme correspond at least to types of leucocytes.

13. The training system (100) according to claim 12, wherein the classes in the classification scheme correspond to types of leucocytes and at least one additional class corresponding to anomalous leucocytes.

14. The training system (100) according to claims 1 to 13, wherein the training images (A1) contain between 10% and 30%, preferably 20%, of modified images (G1, G2, G3, g4, g5, g6, g7, g8).

15. A training method (M) for training an artificial neural network (20), preferably using the training system (100) according to any of the previous claims, comprising the following steps:
acquiring (M1) a set of images (B1);
generating (M2) a set of images based on a modification of the acquired images (B1), wherein each of the modified images (G1, G2, G3, g4, g5, g6, g7, g8) contains less texture information than the respective acquired image (B1, b2, b3) from which they stem;
initializing (M3) an artificial neural network (20); and
training (M4) the artificial neural network (20) with training images (A1) comprising at least part of the modified images (G1, G2, G3, g4, g5, g6, g7, g8) and at least part of the acquired images (B1, b2, b3).
